# EUROPEAN PATENT SPECIFICATION

(11) **EP 3 176 266 B1**
(45) Date of publication and mention of the grant of the patent: **27.02.2019**
(21) Application number: 15826890.4
(22) Date of filing: 30.07.2015
(51) Int. Cl.: C12Q 1/02, C12M 1/26

(54) **METHOD FOR CAPTURING RARE CELLS IN BLOOD**
VERFAHREN ZUR ERFASSUNG VON SELTENEN ZELLEN IN BLUT
PROCÉDÉ DE CAPTURE DE CELLULES RARES DANS LE SANG

(30) Priority: 30.07.2014 US 201462030786 P
(43) Date of publication of application: 07.06.2017
(73) Proprietor: Hitachi Chemical Co., Ltd., Chiyoda-ku Tokyo 100-6606 (JP)
(72) Inventor: TAKAI, Kenji, Tokyo 100-6606 (JP); TSAI, Anthony H., Irvine, CA 92617 (US); YAGI, Satomi, Tokyo 100-6606 (JP); MATSUNAGA, Tatsuya, Tokyo 100-6606 (JP)
(74) Representative: Hoffmann Eitle
(86) International application number: PCT/JP2015/071637
(87) International publication number: WO 2016/017756

(56) References cited:
- WO-A1-2013/158044
- CN-A- 103 725 648
- CN-A- 104 178 454
- JP-A- 2011 505 152
- JP-A- 2013 042 689
- JP-A- 2013 255 487
- US-A1- 2008 057 505
- US-A1- 2014 008 210
- CHANG, CHUN-LI ET AL.: 'Circulating tumor cell detection using a parallel flow micro-aperture chip system' LAB ON A CHIP vol. 15, no. 7, 01 January 2015, pages 1677 - 1688, XP055390823 DOI: 10.1039/C5LC00100E
- CHUNG, YAO-KUANG ET AL.: 'An electrical biosensor for the detection of circulating tumor cells' BIOSENSORS AND BIOELECTRONICS vol. 26, no. 5, 15 January 2011, pages 2520 - 2526, XP027580192

## Description

### Technical Field

The present invention relates to method for capturing rare cells in blood using a filter, which allows for efficient capture of rare cells in blood, particularly circulating tumor cells (hereinafter referred to as CTCs) in blood.

### Background Art

The research/clinical significance of tumor cell concentration is extremely large. If tumor cells in blood can also be concentrated, it can be applied to the diagnosis of a cancer. For example, the most important factor in the prognosis and treatment of a cancer is whether the tumor cells have been metastasized at the time of the first medical examination and during the treatment. In the case where the initial spread of tumor cells has reached the peripheral blood, the detection of CTCs is a useful means for evaluating the progression of a cancer. However, because an overwhelmingly large number of blood components, such as red blood cells and white blood cells, are present in blood, it is difficult to detect CTCs, whose amount is extremely small.

In recent years, a method in which a resin filter using parylene is used to efficiently detect a small amount of CTCs has been proposed (Patent Literature 1).

Alternatively, a method in which a filter using metal in place of resin is used to improve the filter strength, and the cells are separated based on the difference in deformability between blood cells and tumor cells, has also been proposed (Patent Literature 2).

Alternatively, in recent years, a method in which white blood cells are selectively removed, and the remaining cells as rare cells in blood are extracted, has been proposed (Non Patent Literatures 1 to 6).

### Citation List

### Patent Literature

Patent Literature 1: WO 2010/135603
Patent Literature 2: Japanese Unexamined Patent Publication No. 2013-42689

### Non Patent Literature

Non Patent Literature 1: Fehm T, Solomayer EF, Meng S, Tucker T, Lane N, Wang J, et al., "Methods for isolating circulating epithelial cells and criteria for their classification as carcinoma cells", Cytotherapy, 2005; 7: 171-85.
Non Patent Literature 2: Königsberg R, Obermayr E, Bises G, Pfeiler G, Gneist M, Wrba F, et al., "Detection of EpCAM positive and negative circulating tumor cells in metastatic breast cancer patients", Acta Oncol, 2011; 50: 700-10.
Non Patent Literature 3: Sieuwerts AM, Kraan J, Bolt J, van der Spoel P, Elstrodt F, Schutte M, et al., "Anti-epithelial cell adhesion molecule antibodies and the detection of circulating normal-like breast tumor cells", J Natl Cancer Inst, 2009; 101: 61-6.
Non Patent Literature 4: Mostert B, Kraan J, Bolt-de Vries J, van der Spoel P, Sieuwerts AM, Schutte M, et al., "Detection of circulating tumor cells in breast cancer may improve through enrichment with anti-CD146", Breast Cancer Res Treat, 2011; 127: 33-41.
Non Patent Literature 5: Schindlbeck C, Stellwagen J, Jeschke U, Karsten U, Rack B, Janni W, et al., "Immunomagnetic enrichment of disseminated tumor cells in bone marrow and blood of breast cancer patients by the Thomsen-Friedenreich-Antigen", Clin Exp Metastasis, 2008; 25: 233-40.
Non Patent Literature 6: Deng G, Herrler M, Burgess D, Manna E, Krag D, Burke JF, "Enrichment with anti-cytokeratin alone or combined with anti-EpCAM antibodies significantly increases the sensitivity for circulating tumor cell detection in metastatic breast cancer patients", Breast Cancer Res, 2008; 10: R69.

### Summary of Invention

### Technical Problem

According to a filter method, white blood cells, red blood cells, and platelets in blood can be efficiently removed. However, white blood cells are close to tumor cells in size, and thus cannot be entirely removed.

A filter method is a method in which white blood cells and tumor cells are separated based on the difference in size and the difference in deformability. In this method, by tailoring the material of the filter, the pore shape, the pore size, the aperture ratio, and the flow rate, white blood cells and tumor cells can be fairly separated.

However, when the amount of blood is increased, the number of residual white blood cells tends to increase. Usually, a healthy individual possesses 3,500 to 9,500 white blood cells/µL.

CellSearch is the only test system for tumor cells in blood approved by U.S. Food and Drug Administration (FDA), according to which only tumor cells having EpCAM can be selectively captured. However, because only tumor cells having EpCAM can be captured, the clinical significance of this method has been questioned. The amount of blood used in this method is 7.5 mL.

As a result of extensive research by the inventors, in the case where 7.5 mL of blood is allowed to flow, the number of residual white blood cells is about 2,000.

At present, it is believed that the limit of the concentration rate is about 1:25000.

In recent years, the clinical significance of CellSearch has been questioned. That is, it is said that by simply counting the number of tumor cells, only the prognosis of cancer patients can be predicted.

In recent years, a downstream(downstream analysis), such as the gene analysis of collected cells, has been required. In this case, the number of white blood cells acceptable per tumor cell is 10 to 100 (the smaller the better).

In the case where 7.5 mL of blood is allowed to flow, the number of white blood cells acceptable is preferably less than 500, still more preferably less than 100, still more preferably less than 50, and still more preferably less than 10.

That is, the number 2,000 is insufficient considering a downstream.

Meanwhile, although a method in which white blood cells are selectively removed, and the remaining cells as rare cells in blood are extracted, has also been proposed, this method also has its limit.

Also in this method, as a result of extensive research by the inventors, in the case where 7.5 ml of blood is allowed to flow, the number of residual white blood cells is about 2,000. In addition, an attempt to increase the white blood cell removal rate requires the addition of a large amount of magnetic beads, which increases the cost.

Although a large number of CTC removal methods have been proposed in addition to the above methods, each method alone has its limit.

The present invention has been accomplished against the above background. An object thereof is to provide a method for capturing rare cells in blood, according to which white blood cells are reduced using magnetic beads, and then the white blood cells are further reduced using a filter, whereby a sample containing the capture of rare cells in blood suitable for a downstream can be prepared.

### Solution to Problem

A method for capturing rare cells in blood according to the present invention is a method for capturing rare cells in blood, including isolating rare cells in blood from blood, as defined in the appended claims. The method includes a step of removing white blood cells after blood filtration using a filter.

Here, the step of removing white blood cells is a step of removing white blood cells using beads having magnetic properties.

According to one aspect, it is possible that the number of white blood cells in a liquid containing the rare cells in blood obtained after the blood filtration is 10,000 or less.

According to one aspect, it is possible that the method includes, in or after the step of removing white blood cells, a step of diluting a cell suspension with an aqueous solution containing the serum or plasma of a mammal or a protein therefrom.

According to one aspect, it is possible that the serum or plasma of a mammal is of bovine, equine, or human origin.

According to one aspect, it is possible that the serum or plasma of a mammal is from a bovine fetus.

According to one aspect, it is possible that the concentration of the serum or plasma of a mammal in the aqueous solution is within a range of 1% to 50%.

According to one aspect, it is possible that the aqueous solution of serum or plasma contains a phosphate buffer as a main component.

According to one aspect, it is possible that the aqueous solution of serum or plasma contains an anticoagulant.

According to one aspect, it is possible that the anticoagulant is EDTA, heparin, sodium citrate, or sodium fluoride.

According to the invention, the method further includes, before the blood filtration, a step of immersing the filter in an aqueous solution containing the serum or plasma of a mammal.

According to one aspect, it is possible that the method further includes, after the blood filtration and before the step of removing white blood cells, a step of washing blood cells with an aqueous solution containing the serum or plasma of a mammal.

According to one aspect, it is possible that the serum or plasma of a mammal is of bovine, equine, or human origin.

According to one aspect, it is possible that the serum of a mammal is fetal bovine serum or plasma.

According to the invention, the filter has a surface made of gold, platinum, palladium, or an alloy thereof.

According to one aspect, it is possible that the filter contains nickel as a main component and has a surface plated with gold, platinum, palladium, or an alloy thereof.

According to one aspect, it is possible that the filter contains copper as a main component and has a surface plated with gold, platinum, palladium, or an alloy thereof.

According to one aspect, it is possible that the filter contains palladium as a main component and has a surface plated with gold, platinum, or an alloy thereof.

According to one aspect, it is possible that an outermost layer of the filter is gold plating.

According to one aspect, it is possible that the surface of gold, platinum, palladium or an alloy thereof is a plating having a thickness of 0.05 µm to 1 µm.

According to one aspect, it is possible that the rare cells in blood are tumor cells.

According to one aspect, it is possible that the filter has through pores whose opening shape is a circle, an ellipse, a rounded-corner rectangle, a rectangle, or a square. Incidentally, a rounded-corner rectangle is a shape composed of two longer sides of equal length and two semicircles. In the case where the opening shape is a rectangle or a rounded-corner rectangle, the through pores are less likely to be clogged, and the concentration rate of the component to be captured can be further improved.

According to one aspect, it is possible that the filter has through pores whose shape is at least one of a rectangle and a rounded-corner rectangle, the minor length thereof being 5 µm to 15 µm.

According to one aspect, it is possible that the filter has a thickness of 3 µm to 50 µm.

### Advantageous Effects of Invention

According to the present invention, a method for capturing rare cells in blood, which allows for the preparation of a sample containing the capture of rare cells in blood suitable for a downstream, is provided.

### Brief Description of Drawings

FIG 1 illustrates the schematic structure of a cell capture cartridge.
FIG 2 illustrates a method for producing a filter.
FIG 3 is a schematic cross-sectional view showing a method for producing a filter using a copper plate (or an Ni plate in the case of copper plating).
FIG. 4 is a diagram illustrating the definition of the major pore size, minor pore size, and aperture ratio in a filter.

### Description of Embodiments

Hereinafter, modes for carrying out the present invention will be described in detail with reference to the attached drawings. Incidentally, in the description of drawings, same elements will be indicated with same symbols, and redundant description will be omitted.

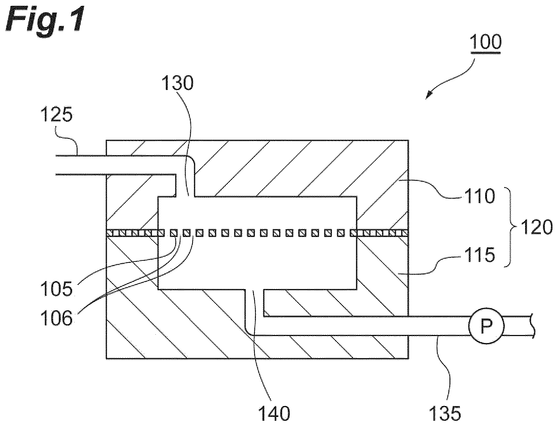

In the method for capturing rare cells in blood according to this embodiment, rare cells in blood are filtered from blood using a filter. The configuration of the device for blood filtration is not particularly limited. As an example, the case of using a cell capture cartridge will be described. The cell capture cartridge 100 includes, as shown in FIG. 1: a casing 120 that has an inflow port 130 having connected thereto an inflow tube 125 in which a cell dispersion flows and an outflow port 140 having connected thereto an outflow tube 135 from which a cell dispersion flows; and a filter 105 that is located in the casing 120 and has a capturing region for capturing rare cells in blood contained in a cell dispersion.

The casing 120 is a member for holding the filter 105 and composed of an upper member 110 and a lower member 115. The shape of the casing 120 is not particularly limited and may be a rectangular parallelepiped, a circular cylinder, or the like.

Blood, a processing liquid such as a washing liquid, or the like, for example, is connected upstream the inflow tube 125. In addition, a pump P is connected downstream the outflow tube 135. Accordingly, when the pump P is driven, blood or the like is supplied from the inflow tube 125 into the casing 120 and then discharged outside from the outflow tube 135.

The filter 105 has formed therein through pores 106. When blood is introduced into the cell capture cartridge 100, although the red blood cells and the like of blood pass through the through pores 106, rare cells in blood cannot pass through the through pores 106 and remain on the filter 105 surface. As a result, rare cells in blood can be recovered.

Incidentally, the shape of the cell capture cartridge 100 is not limited to the above. As long as rare cells in blood can be captured by blood filtration using a filter, the device configuration is not particularly limited.

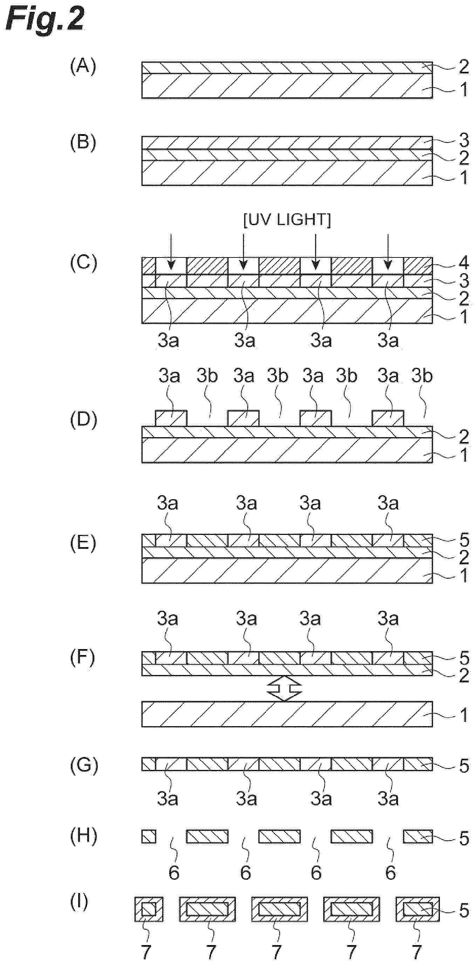

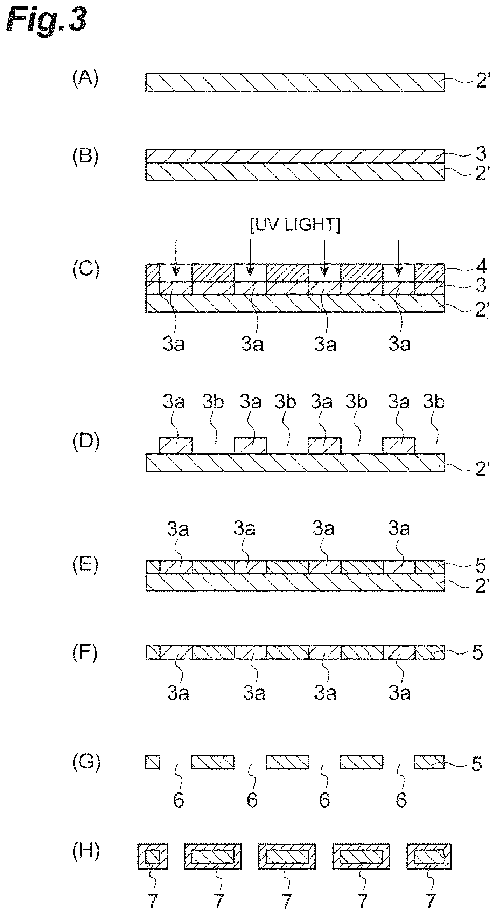

Next, with reference to FIG. 2 and FIG 3, an example of a filter preparation method will be shown, and also the filter will be described.

FIG. 2 is a schematic cross-sectional view showing a method for producing a metal thin-film filter using a substrate composed of a peelable copper foil (or an Ni foil in the case of copper plating) attached to an MCL.

FIG. 2(A) shows a substrate including an MCL 1 and a peelable copper foil 2 (or an Ni foil in the case of copper plating) laminated thereon. This substrate is prepared first.

It is preferable that the material used for the substrate for producing a filter is copper (or nickel in the case of copper plating). Copper is easily removable by chemical lysis using a liquid chemical, and is also superior to other materials in terms of adhesion to a photoresist.

Next, as shown in FIG. 2(B), a photoresist 3 is formed on the peelable copper foil 2 of the substrate. It is preferable that the thickness of the photoresist is 1.0 to 2.0 times the thickness of a conductor to be formed later. When the thickness is small, it becomes difficult to strip the resist later, while when the thickness is large, it becomes difficult to form a circuit. Specifically, it is preferable that the photoresist 3 has a thickness of 15 to 50 µm. As a photosensitive resin composition for forming the photoresist 3, a negative type photosensitive resin composition is preferable. As the negative type photosensitive resin composition, a resin composition containing at least a binder resin, a photopolymerizable compound having an unsaturated bond, and a photopolymerization initiator is preferable.

Next, as shown in FIG 2(C), a photomask 4 is placed to cover the photoresist 3, and then the photoresist is exposed to light. As a result, an exposed area 3a is formed in the region that is not covered with the photomask 4.

Next, as shown in FIG 2(D), the photoresist is developed with an alkaline solution or the like to remove the unexposed area 3b. As a result, the exposed area 3a remains at the positions corresponding to through pores of a filter.

Next, as shown in FIG. 2(E), the portion that is not covered with the photoresist is subjected to electroplating to form a plating layer 5. This plating portion serves as the material of a filter.

Next, as shown in FIG 2(F), the peelable copper foil 2 having the plating layer 5 formed thereon by electroplating is stripped from the MCL 1.

Next, as shown in FIG. 2(G), the peelable copper foil 2 is removed by chemical lysis with a liquid chemical, and the self-supported film formed of the exposed area 3a and the plating layer 5 is isolated.

Next, as shown in FIG. 2(H), the exposed area 3a of the photoresist remaining in the self-supported film is removed, whereby through pores 6 are formed in the plating layer 5.

Next, as shown in FIG. 2(I), electroless gold plating is performed to form a gold plating layer 7 on the filter surface. As a result, a filter 105 for use in the cell capture cartridge 100 can be produced.

FIG. 3 is a schematic cross-sectional view showing a method for producing a metal thin-film filter using a copper plate (or an Ni plate in the case of copper plating).

The method shown in FIG. 3 is the same as the production method shown in FIG. 2 except for that a copper plate (or an Ni plate) 2' is used in place of the MCL 1 and the peelable copper foil 2 in the method shown in FIG 2.

That is, FIG. 3(A) shows a step of preparing a copper plate 2' for use as a substrate.

In addition, FIG. 3(B) shows a step of laminating a photoresist 3 on the copper plate 2'. In addition, FIG. 3(C) shows the exposure of the photoresist to light through a photomask 4. In addition, FIG 3(D) shows the development of the photoresist to remove the unexposed area 3b. In addition, FIG 3(E) shows a step of electroplating a portion that is not covered with the exposed area 3a of the photoresist, thereby forming a plating layer. In addition, FIG. 3(F) shows a step of removing the copper plate 2' by chemical lysis with a liquid chemical (chemical etching), thereby isolating a self-supported film formed of the exposed area 3a and the plating layer 5. In addition, FIG. 3(G) shows a step of removing the exposed area 3a of the photoresist remaining in the self-supported film, thereby forming through pores 6 in the plating layer 5. In addition, FIG 3 (H) shows a step of electroless gold plating, thereby forming a gold plating layer 7 on the filter surface.

As described above, a filter 105 for use in the cell capture cartridge 100 can be produced by either of the method shown in FIG. 2 and the method shown in FIG. 3.

It is preferable that the material of the filter 105 is a metal. Because metals have excellent processability, the filter processing accuracy can be enhanced. As a result, the capture rate of the component to be captured can be further improved. In addition, metals are more rigid compared with other materials, such as plastics. Accordingly, even when external force is applied, the size and shape can be maintained. Therefore, in the case where a component slightly larger than the through pores are deformed and passed through the filter, more accurate separation/concentration can be achieved.

In addition, it is preferable that the main metal component is nickel, silver, palladium, copper, iridium, or an alloy thereof. These metals are electroplated with gold, platinum, palladium or an alloy thereof.

Palladium and iridium have excellent characteristics in that the oxidation-reduction potential is high and the solubility is low, but are disadvantageous in that they are expensive. Nickel has a lower oxidation-reduction potential than hydrogen and thus is easy to dissolve, but is inexpensive. Silver and palladium are noble metals, and they are relatively inexpensive compared with iridium.

It is preferable that the material used for the substrate (copper plate 2': see FIG. 3) for producing a filter is copper (or nickel in the case where the material of the filter 105 is copper). Copper is easily removable by chemical lysis using a liquid chemical, and is also superior to other materials in terms of adhesion to a photoresist. Incidentally, also as the metal foil 2 (see FIG 2) laminated on the MCL 1, copper (or nickel in the case where the material of the filter 105 is copper) is preferable.

In addition, the formation of the plating layer 5 shown in FIG. 2(E) and FIG 3(E) is performed by electroplating. For example, for nickel electroplating (which is not part of the invention), a Watt bath (containing nickel sulfate, nickel chloride, and boric acid as main components), a sulfamic acid bath (containing nickel sulfamate and boric acid as main components), a strike bath (containing nickel chloride and hydrogen chloride as main components), and the like can be mentioned.

For silver electroplating (which is not part of the invention), a bath containing potassium argentocyanide or potassium tartrate as a main component can be mentioned.

For palladium electroplating, a bath containing a water-soluble palladium salt and a naphthalenesulfonic acid compound can be mentioned.

For iridium electroplating (which is not part of the invention), a bath containing a halogen-containing soluble iridium salt and an alcohol can be mentioned.

For copper electroplating (which is not part of the invention), a bath containing copper sulfate, sulfuric acid, and chloride ions as main components can be mentioned.

Electroplating is performed using such a plating bath. It is suitable that the current density during electroplating is within a range of 0.3 to 4 A/dm², more preferably within a range of 0.5 to 3 A/dm². When the current density is 4 A/dm² or less, roughening can be suppressed, while when the current density is 0.3 A/dm² or more, crystal grains of the metal grow sufficiently, enhancing the effect as a barrier layer. As a result, the effect of this embodiment can be obtained well.

The positions of the resist at the time of plating, that is, the positions of the exposed area 3a, correspond to the positions of through pores. As the opening shape of the through pores, a circle, an ellipse, a square, a rectangle, a rounded-corner rectangle, a polygon, and the like can be mentioned, for example. In terms of efficiently capturing the target component, a circle, a rectangle, and a rounded-corner rectangle are preferable. In addition, in terms of preventing the filter from clogging, a rounded-corner rectangle is particularly preferable.

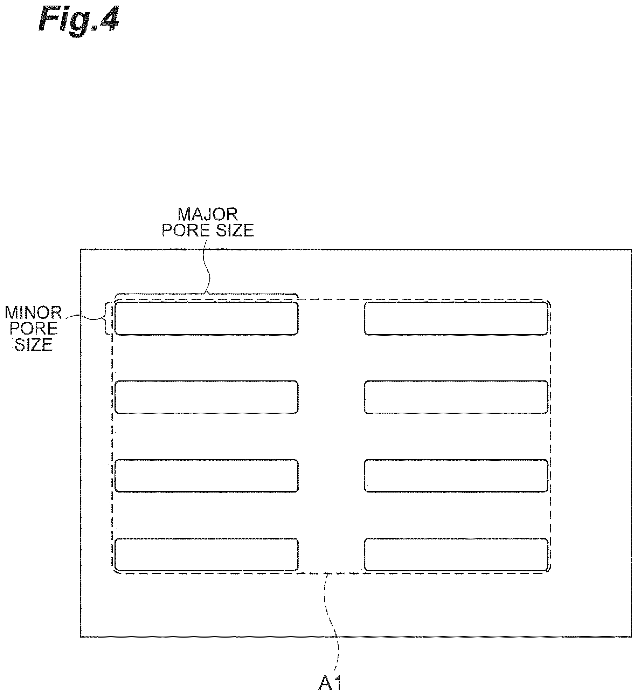

The pore size of the through pores 6 is set according to the size of the component to be captured. As used herein, in the case where the opening shape is not a circular shape, such as the case of an ellipse, a rectangle, or a polygon, the pore size means the maximum diameter of a sphere that can pass through each through pore. For example, in the case where the opening shape is a rectangle, as shown in FIG. 4, although the minor pore size on the shorter side and the major pore size on the longer side can be defined, the minor pore size is used as the pore size of the through pore 6. In addition, in the case where the opening shape is a polygon, the diameter of an inscribed circle of the polygon is used. In the case where the opening shape is a rectangle or a rounded-corner rectangle, even in the state where the component to be captured is captured in the through pore 6, the opening has a gap in the longitudinal direction of the opening shape. A liquid can pass through this gap, and thus the filter can be prevented from clogging. The length of the minor pore size of the filter is preferably 5 µm to 15 µm, and still more preferably 7 µm to 9 µm.

The average aperture ratio of the through pores of the filter 105 is preferably 3 to 50%, more preferably 3 to 20%, and particularly preferably 3 to 10%. Here, an aperture ratio means the proportion of the area occupied by the through pores relative to the area of the region that functions as a filter (see FIG. 4). That is, the area of the region that functions as a filter means the region defined by connecting the outermost parts of through pores among a plurality of through pores contained in the filter (in FIG. 4, region A1 surrounded by the broken line). When the aperture ratio is too high, the pressure on white blood cells decreases, resulting in an increase in the number of residual white blood cells. When the aperture ratio is low, the amount of blood that can flow decreases.

The thickness of the filter 105 is preferably 3 µm to 50 µm, more preferably 5 µm to 30 µm, and particularly preferably 8 µm to 20 µm. In the case where the thickness of the filter 105 is less than 3 µm, the strength of the filter may decrease, resulting in poor handleability. Conversely, when the thickness is more than 50 µm, it is feared that the productivity may decrease due to the increased processing time, the materials may be overconsumed, causing cost disadvantages, or microprocessing itself may become difficult. Further, it becomes difficult for white blood cells to pass therethrough.

After the above circuit formation, the resin layer is stripped, and the copper foil is etched (FIG. 2) or the copper plate is removed (FIG. 3). As a result, as shown in FIG. 2(H) or FIG 3(G), a filter composed of the plating layer 5 is completed.

Next, the resist remaining in the filter (exposed area 3a) is removed with a strong alkali. As the strong alkali, a 0.1 to 10 wt% NaOH or KOH aqueous solution is preferable. In order to promote stripping, monoethanolamine (1 to 20 vol%) or the like may be added. In the case where stripping is difficult, the resist may also be removed with a solution prepared by adding an alkali (0.1 to 10 wt% NaOH or KOH) to sodium permanganate, potassium permanganate, or the like.

The filter from which the resist has been removed is subjected to noble metal plating with gold, platinum, palladium or an alloy thereof.

Among metals for noble metal plating, gold has the highest oxidation-reduction potential among all metals as described above, and is believed to have no cytotoxicity. In addition, almost no discoloration, for example, occurs during long-term storage.

Gold plating may be performed as electroless plating or electroplating. In the case of electroplating, the variation in thickness is likely to increase, affecting the pore size accuracy of the filter. Therefore, electroless plating is desirable. However, gold electroplating can improve the coverage.

Although gold plating by displacement plating alone is also effective, the combination of displacement plating and reduction plating is more effective.

The metal filter before gold plating may have an oxidized surface. Thus, the removal of an oxide film is performed. Here, it is suitable that the filter is washed with an aqueous solution containing a compound that forms a complex with metal ions.

Specifically, an aqueous solution containing a cyan, an EDTA, or a citric acid is suitable.

Among them, citric acids are optimal for the pretreatment before gold plating. Specifically, citric acid anhydride, hydrates of citric acid, citrates, and hydrates of citrates are suitable. Specifically, citric acid anhydride, citric acid monohydrate, sodium citrate, potassium citrate, and the like may be used. Its concentration is preferably within a range of 0.01 mol/L to 3 mol/L, more preferably 0.03 mol/L to 2 mol/L, and particularly preferably 0.05 mol/L to 1 mol/L. When the concentration is 0.01 mol/L or more, the adhesion between the electroless gold plating layer and the metal filter is improved. In addition, a concentration of more than 3 mol/L does not improve the effect and is also economically undesirable.

It is suitable that immersion in the solution containing citric acid is performed at 70°C to 95°C for 1 to 20 minutes.

Within a range where the effects of the present invention can be obtained, the solution containing citric acid may also contain a reducing agent, which is contained in the plating solution or the like, or a buffer such as a pH adjuster. However, it is desirable that the amounts of reducing agent, pH adjuster, and the like are small, and an aqueous solution of citric acid alone is most preferable. The pH of the solution containing citric acid is preferably 5 to 10, and more preferably 6 to 9.

The pH adjuster is not particularly limited as long as it is an acid or an alkali. Examples of usable acids include hydrochloric acid, sulfuric acid, and nitric acid, and examples of alkalis include a solution of a hydroxide of an alkali metal or an alkaline earth metal, such as sodium hydroxide, potassium hydroxide, or sodium carbonate. As described above, they may be used within a range where the effect of citric acid is not inhibited. In addition, when the solution containing citric acid contains nitric acid at a high concentration of 100 mL/L, the adhesion-improving effect decreases as compared with the case of treatment with a solution containing only citric acid.

The reducing agent is not particularly limited as long as it has reducing ability, and examples thereof include hypophosphorous acid, formaldehyde, dimethylamine borane, and sodium borohydride.

Next, substitution gold plating is performed. Substitution gold plating may be performed using a cyanide bath or a non-cyanide bath. Considering the environmental impact or the cytotoxicity of the residue, a non-cyanide bath is desirable. Examples of gold salts contained in a non-cyanide bath include chloraurates, gold sulfite salts, gold thiosulfate salts, and gold thiomalate salts. The gold salts may be used alone, and it is also possible to use a combination of two or more kinds.

Further, the metal-dissolving effect of a cyanide-based bath is too strong, and some metals are likely to be dissolved, forming pinholes. In the case where the pretreatment is sufficiently performed as described above, a non-cyanide plating bath is preferable.

As the supply source of gold, gold sulfites are particularly preferable. As gold sulfites, gold sodium sulfite, gold potassium sulfite, gold ammonium sulfite, and the like are suitable.

It is preferable that the gold concentration is within a range of 0.1 g/L to 5 g/L. When the concentration is less than 0.1 g/L, gold is less likely to be deposited, while when it is more than 5 g/L, the solution is likely to be decomposed.

It is suitable that the substitution gold plating bath contains an ammonium salt or an ethylenediaminetetraacetic acid salt as a gold complexing agent. Examples of ammonium salts include ammonium chloride and ammonium sulfate, and examples of ethylenediaminetetraacetic acid salts include ethylenediaminetetraacetic acid, sodium ethylenediaminetetraacetate, potassium ethylenediaminetetraacetate, and ammonium ethylenediaminetetraacetate. It is preferable that the ammonium salt is used at a concentration within a range of 7 × 10⁻³ mol/L to 0.4 mol/L. When the ammonium salt concentration is outside of this range, the solution tends to be unstable. It is preferable that the ethylenediaminetetraacetic acid salt is used at a concentration within a range of 2 × 10⁻³ mol/L to 0.2 mol/L. When the ammonium salt concentration is outside of this range, the solution tends to be unstable.

In order to keep the solution stable, it is suitable that a sulfurous acid salt is contained at 0.1 g/L to 50 g/L. Examples of sulfurous acid salts include sodium sulfite, potassium sulfite, and ammonium sulfite.

As a pH adjuster, in the case where the pH is to be reduced, it is preferable to use hydrochloric acid or sulfuric acid. In addition, in the case where the pH is to be increased, it is preferable to use sodium hydroxide, potassium hydroxide, or aqueous ammonia. It is suitable that the pH is adjusted to 6 to 7. A pH outside of this range adversely affects the stability of the solution or the appearance of the plating.

It is preferable that displacement plating is used at a solution temperature of 30°C to 80°C. A pH outside of this range adversely affects the stability of the solution or the appearance of the plating.

Displacement plating is performed as described above. However, it is difficult to completely cover the metal by displacement plating. Thus, next, reduction-type gold plating using a reducing agent is performed. It is preferable that the thickness of the displacement plating is within a range of 0.02 µm to 0.1 µm.

As gold salts for reduction-type gold plating, gold sulfite salts and gold thiosulfate salts are preferable, and it is preferable that the content thereof is, as gold, within a range of 1 g/L to 10 g/L. When the gold content is less than 1 g/L, the gold deposition reaction decreases, while when it is more than 10 g/L, the stability of the plating solution decreases, and also the consumption of gold increases due to the loss of the plating solution; therefore, this is undesirable. It is more preferable that the content is 2 g/L to 5 g/L.

Examples of reducing agents include hypophosphoric acid, formaldehyde, dimethylamine borane, and sodium borohydride, but phenyl compound-based reducing agents are more preferable. Examples thereof include phenol, o-cresol, p-cresol, o-ethylphenol, p-ethylphenol, t-butylphenol, o-aminophenol, p-aminophenol, hydroquinone, catechol, pyrogallol, methyl hydroquinone, aniline, o-phenylene diamine, p-phenylene diamine, o-toluidine, o-ethylaniline, and p-ethylaniline. They may be used alone, and it is also possible to use two or more kinds.

It is preferable that the reducing agent content is 0.5 g/L to 50 g/L. When the reducing agent content is less than 0.5 g/L, it tends to be difficult to obtain a practical deposition rate, while when it is more than 50 g/L, the stability of the plating solution tends to decrease. The reducing agent content is more preferably 2 g/L to 10 g/L, and particularly preferably 2 g/L to 5 g/L.

The electroless gold plating solution may contain a heavy metal salt. In terms of promoting the deposition rate, it is preferable that the heavy metal salt is at least one member selected from the group consisting of thallium salts, lead salts, arsenic salts, antimony salts, tellurium salts, and bismuth salts.

Examples of thallium salts include inorganic compound salts, such as thallium sulfate, thallium chloride, thallium oxide salt, and thallium nitrate, and organic complex salts, such as dithallium malonate. Examples of lead salts include inorganic compound salts, such as lead sulfate and lead nitrate, and organic acetic acid salts, such as acetate.

In addition, examples of arsenic salts include inorganic compound salts and organic complex salts, such as arsenite, arsenate, and arsenic trioxide. Examples of antimony salts include organic complex salts, such as antimonyl tartrate, and inorganic compound salts, such as antimony chloride, antimony oxysulfate, and antimony trioxide.

Examples of tellurium salts include inorganic compound salts and organic complex salts, such as tellurite and tellurate. Examples of bismuth salts include inorganic compound salts, such as bismuth(III) sulfate, bismuth(III) chloride, and bismuth(III) nitrate, and organic complex salts, such as bismuth (III) oxalate.

One or more kinds of the above heavy metal salts may be used. Their total amount added is preferably 1 ppm to 100 ppm, more preferably 1 ppm to 10 ppm, based on the total volume of the plating solution. When the amount is less than 1 ppm, the improving effect on the deposition rate may not be sufficient, while when it is more than 100 ppm, the plating solution stability tends to decrease.

The electroless gold plating solution may contain a sulfur-based compound. When a sulfur compound is further contained in the electroless gold plating solution containing a phenyl compound-based reducing agent and a heavy metal salt, a sufficient deposition rate can be obtained even when the solution temperature is as low as 60 to 80°C, and also excellent film appearance can be achieved. Moreover, the stability of the plating solution is particularly improved.

Examples of sulfur-based compounds include sulfide salts, thiocyanates, thiourea compounds, mercaptan compounds, sulfide compounds, disulfide compounds, thioketone compounds, thiazole compounds, and thiophene compounds.

Examples of sulfide salts include potassium sulfide, sodium sulfide, sodium polysulfide, and potassium polysulfide, examples of thiocyanates include sodium thiocyanate, potassium thiocyanate, and potassium dithiocyanate, and examples of thiourea compounds include thiourea, methylthiourea, and dimethylthiourea.

Examples of mercaptan compounds include 1,1-dimethylethanethiol, 1-methyl-octanethiol, dodecanethiol, 1,2-ethanedithiol, thiophenol, o-thiocresol, p-thiocresol, o-dimercaptobenzene, m-dimercaptobenzene, p-dimercaptobenzene, thioglycol, thiodiglycol, thioglycolic acid, dithioglycolic acid, thiomalic acid, mercaptopropionic acid, 2-mercaptobenzimidazole, 2-mercapto-1-methylimidazole, and 2-mercapto-5-methylbenzimidazole.

Examples of sulfide compounds include diethyl sulfide, diisopropyl sulfide, ethyl isopropyl sulfide, diphenyl sulfide, methylphenyl sulfide, rhodanine, thiodiglycolic acid, and thiodipropionic acid, and examples of disulfide compounds include dimethyl disulfide, diethyl disulfide, and dipropyl disulfide.

Further, examples of thioketone compounds include thiosemicarbazide, examples of thiazole compounds include thiazole, benzothiazole, 2-mercaptobenzothiazole, 6-ethoxy-2-mercaptobenzothiazole, 2-aminothiazole, 2,1,3-benzothiadiazole, 1,2,3-benzothiadiazole, (2-benzothiazolythio)acetic acid, and 3-(2-benzothiazolythio)propionic acid, and examples of thiophene compounds include thiophene and benzothiophene.

Sulfur-based compound may be used alone, and it is also possible to use two or more kinds. The sulfur-based compound content is preferably 1 ppm to 500 ppm, more preferably 1 ppm to 30 ppm, and particularly preferably 1 ppm to 10 ppm. When the sulfur-based compound content is less than 1 ppm, the deposition rate decreases, resulting in poor plating coverage, whereby the film appearance is deteriorated. When the content is more than 500 ppm, it becomes difficult to control the concentration, making the plating solution unstable.

The electroless gold plating solution preferably contains, in addition to the gold salt, reducing agent, heavy metal salt, and sulfur-based compound described above, at least one of a complexing agent, a pH buffer, and a metal ion masking agent, and more preferably contains all of them.

It is preferable that the electroless gold plating solution of the present invention contains a complexing agent. Specific examples thereof include non-cyanide complexing agents such as sulfites, thiosulfates, and thiomalates. It is preferable that the complexing agent content is 1 g/L to 200 g/L based on the total volume of the plating solution. In the case where the complexing agent content is less than 1 g/L, the gold complexing ability decreases, resulting in a decrease in stability. When the content is more than 200 g/L, although the plating stability is improved, recrystallization occurs in the solution, which is economically not good. It is more preferable that the complexing agent content is 20 g/L to 50 g/L.

It is preferable that the electroless gold plating solution contains a pH buffer. A pH buffer is effective in maintaining the deposition rate constant to stabilize the plating solution. It is also possible to mix a plurality of buffers. Examples of pH buffers include phosphates, acetates, carbonates, borates, citrates, and sulfates. Among them, boric acid and sulfates are particularly preferable.

It is preferable that the pH buffer content is 1 g/L to 100 g/L based on the total volume of the plating solution. When the pH buffer content is less than 1 g/L, no pH-buffering effect is obtained, while when it is more than 100 g/L, recrystallization may occur. It is more preferable that the content is 20 g/L to 50 g/L.

It is preferable that the gold plating solution contains a masking agent. As the masking agent, a benzotriazole-based compound can be used. Examples of benzotriazole-based compounds include benzotriazole sodium, benzotriazole potassium, tetrahydrobenzotriazole, methylbenzotriazole, and nitrobenzotriazole.

It is preferable that the metal ion masking agent content is 0.5 g/L to 100 g/L based on the total volume of the plating solution. When the metal ion masking agent content is less than 0.5 g/L, the masking effect on impurities is low, and there is a tendency that the solution stability cannot be sufficiently secured. Meanwhile, when the content is more than 100 g/L, recrystallization may occur in the plating solution. In terms of cost and effect, the most preferable range is 2 g/L to 10 g/L.

It is preferable that the gold plating solution has a pH within a range of 5 to 10. In the case where the pH of the plating solution is less than 5, the sulfite or thiosulfate, which serves as a complexing agent in the plating solution, may be decomposed, resulting in the generation of toxic sulfurous acid gas. In the case where the pH is more than 10, the stability of the plating solution tends to decrease. In order to improve the deposition efficiency of the reducing agent and obtain a high deposition rate, it is preferable that the pH of the electroless gold plating solution is within a range of 8 to 10.

As a method for electroless plating, the filter after substitution gold plating is immersed and subjected to gold plating.

It is suitable that the plating solution temperature is 50°C to 95°C. When the temperature is less than 50°C, the deposition efficiency is low, while when it is 95°C or more, the solution is likely to be unstable.

It is preferable that the gold plating layer 7 formed in this manner, which is the outermost layer, has a gold purity of 99 wt% or more. When the gold purity of the gold plating layer 7 is less than 99 wt%, the contact portion has increased cytotoxicity. In terms of enhancing the reliability, it is more preferable that the purity of the gold layer is 99.5 wt% or more.

In addition, the thickness of the gold plating layer 7 is preferably 0.005 µm to 3 µm, more preferably 0.05 µm to 1 µm, and still more preferably 0.1 µm to 0.5 µm. When the thickness of the gold plating layer 7 is 0.005 µm or more, metal elution can be suppressed to some extent. Meanwhile, even when the thickness is more than 3 µm, the effect is not improved any further, and, therefore, also from an economical point of view, it is preferable that the thickness is 3 µm or less. In the case of noble metal plating, it is preferable that the thickness is 0.05 µm to 1 µm.

The gold surface formed as described above has no cytotoxicity, and is stable in air or in most blood-containing aqueous solutions. However, the gold surface is relatively hydrophobic and has low biocompatibility, and thus it is suitable to subject the surface to a biocompatibility-improving treatment. An example of the surface treatment will be shown hereinafter.

White blood cells, red blood cells, and platelets, which are components in blood, show a rejection response to foreign substances. Therefore, the metal surface is pretreated. In this case, it is preferable that the metal surface is immersed in a solvent containing a biocompatible polymer immediately before blood filtration.

In the present invention, solvent containing a biocompatible polymer is an aqueous solution containing the serum or plasma of a mammal. In the case where blood is collected in a blood collection tube containing an anticoagulant and then allowed to stand or be centrifuged, the supernatant resulting from the precipitation of blood cell components is plasma. Such plasma contains a coagulation factor. Meanwhile, in the case where blood is collected in a blood collection tube containing no anticoagulant and then allowed to stand, because there is no anticoagulant, the blood cell components coagulate due to a coagulation factor. The supernatant in that case is serum, and serum lacks a coagulation factor.

Among mammals, considering the characteristics or price, it is preferable that the mammal is bovine, equine, or human, and fetal bovine serum is particularly preferable.

Blood serum contains proteins. Albumin or globulin contained in proteins is adsorbed on the filter surface, whereby blood cell components are prevented from being adsorbed on the filter surface. Proteins are abundantly present in serum, and serum albumin occupies about 50% to 65%.

Albumin contains a large number of amino acids connected, and thus has a large number of amino groups. An amino group firmly coordinates with a noble metal (gold, platinum, palladium).

Particularly in the case of gold, because an oxide film is hardly present, firm binding to albumin is formed even without any special pretreatment.

The filter 105 is pretreated with an aqueous solution prepared by diluting such serum or plasma. The concentration of serum or plasma is preferably within a range of 1% to 50%, still more preferably within a range of 5% to 30%, and still more preferably within a range of 10% to 20%.

It is suitable that serum or plasma is diluted with a water-based solvent, and it is desirable that a buffer, such as phosphoric acid, is contained. It is preferable that the aqueous solution of serum or plasma contains a phosphate buffer as a main component.

Further, it is desirable that the serum or plasma solution contains an anticoagulant of blood. Examples of such anticoagulants include EDTA, heparin, sodium citrate, and sodium fluoride. Among them, EDTA or heparin is desirable.

The treatment time is preferably 1 minute or more and 60 minutes or less, and still more preferably 1 minute or more and 10 minutes or less. In the case where the time is less than 1 minute, the biocompatible polymer is less likely to firmly coordinate with the noble metal surface, while a treatment for more than 60 minutes is undesirable in terms of working hours.

Blood is passed through the filter treated with a biocompatible polymer in this manner (blood filtration). The filter 105 is installed to the cell capture cartridge 100 or a like device, and blood is passed therethrough. The blood may be treated from below the filter with a negative pressure, treated from above the filter with a positive pressure, or treated with a centrifugal force as in centrifugation. In any method, it is important to control the linear velocity at which blood passes through the pores of the filter.

Incidentally, the amount of blood necessary as a sample is 1 to 10 ml.

It is preferable that the blood collection tube for collecting blood contains an anticoagulant. Examples thereof include EDTA-2K, EDTA-2Na, sodium citrate, sodium fluoride, and heparin.

In addition, a method in which blood collected in a blood collection tube is entirely used as a sample and a method in which only the buffy coat is isolated by centrifugation are possible. Although the method in which blood collected in a blood collection tube is entirely used is easier, the removal of red blood cells and like components is advantageous in that the subsequent steps are facilitated.

In the case where the method in which only the buffy coat is isolated is used, it is suitable that dilution is performed with a solvent containing a biocompatible polymer. As a result, the adsorption of tumor cells in blood at unnecessary positions, for example, can be prevented.

Examples of solvents containing a biocompatible polymer include the serum and plasma of a mammal. In the case where blood is collected in a blood collection tube containing an anticoagulant and then allowed to stand or be centrifuged, the supernatant resulting from the precipitation of blood cell components is plasma. Such plasma contains a coagulation factor. Meanwhile, in the case where blood is collected in a blood collection tube containing no anticoagulant and then allowed to stand, because there is no anticoagulant, the blood cell components coagulate due to a coagulation factor. The supernatant in that case is serum, and serum lacks a coagulation factor.

Among mammals, considering the characteristics or price, it is desirable that the mammal is bovine, equine, or human. In particular, fetal bovine serum is likely to offer excellent characteristics.

Blood serum contains proteins. Albumin or globulin contained in proteins is adsorbed on the filter surface, whereby blood cell components are prevented from being adsorbed on the filter surface. Proteins are abundantly present in serum, and serum albumin occupies about 50% to 65%.

The filter is pretreated with a solution prepared by diluting such serum or plasma. The concentration of serum or plasma is preferably within a range of 1% to 50%, still more preferably within a range of 5% to 30%, and still more preferably within a range of 10% to 20%.

It is suitable that serum or plasma is diluted with a water-based solvent, and it is desirable that a buffer, such as phosphoric acid, is contained.

Further, it is desirable that the serum or plasma solution contains an anticoagulant of blood. Examples of such anticoagulants include EDTA, heparin, sodium citrate, and sodium fluoride. Among them, EDTA or heparin is desirable.

After a cell suspension containing white blood cells and CTCs diluted with a biocompatible polymer-containing solvent is prepared in this manner, white blood cells are removed.

For the removal of white blood cells, a white blood cell removal method using magnetic beads (beads having magnetic properties), which is a conventionally known method, is used. As such beads, Dynabeads M-450-CD45 (pan-leucocyte) can be mentioned.

The above magnetic beads are allowed to react with a CD45 antibody, and then immersed in the cell suspension. At this time, it is suitable that the amount of magnetic beads is about four to ten times the amount of white blood cells. When the amount of magnetic beads is small, the white blood cell removal efficiency decreases, while when the amount of magnetic beads is large, it leads to increased cost.

Magnetic beads and white blood cells are allowed to react for a predetermined period of time, and then the magnetic beads are removed.

Through the above step, the number of white blood cells can be reduced from the original order of 50,000,000 (in the case of 5 mL) to at least the order of 500,000.

The suspension of cells obtained through the above step is subjected to blood filtration, whereby reduction is made to the level that allows for a downstream.

The linear velocity at which blood passes through pores of the filter 105 (volume of blood/total area of pores) is desirably within a range of 0.5 to 100 cm/min, and still more desirably within a range of 5 to 20 cm/min.

It is suitable that the filter 105 after blood filtration is washed again with a serum or plasma solvent. As the serum or plasma solvent, one completely the same as the solvent used for immersion described above can be used. The linear velocity of the washing liquid is also desirably within a range of 0.5 to 100 cm/min, and still more desirably within a range of 5 to 20 cm/min.

Because of the immersion in the beginning, the biocompatible polymer has been adsorbed on the surface of the filter 105, and thus the adsorption of blood cell components during filtration can be suppressed. However, as a result of the blood treatment, blood cells become likely to be adsorbed on the surface.

In addition, when the biocompatible polymer is allowed to flow at the time of washing again, the washing effect is enhanced.

Although the amount of washing liquid may be suitably adjusted, at the above linear velocity, washing for 1 to 20 minutes is desirable, and washing for 5 to 15 minutes is still more desirable.

The concentrated rare cells, such as CTCs, can be captured as above. When the filter is chemically firmly coated with a biocompatible polymer, components such as red blood cells, white blood cells, and platelets can be eliminated. Incidentally, it is necessary to include at least either of a step of immersing the filter in an aqueous solution containing the serum or plasma of a mammal before blood filtration and a step of washing blood cells of the blood with an aqueous solution containing the serum or plasma of a mammal after blood filtration. However, particularly when the treatment is performed before blood filtration, rare cells in blood can be efficiently captured.

In a filter method using blood filtration, the amount of eventually remaining white blood cells tends to vary depending on the amount of white blood cells initially added. According to the inventors' examination, the amount of white blood cells can be reduced to about 1/25000 by filtration through a filter.

However, in some cases, about 50,000,000 white blood cells may be present in 7.5 ml of blood. In such a case, about 2,000 (1,000 to 10,000) white blood cells will remain.

Even when there are 2,000 white blood cells remaining, it is possible to count tumor cells. However, in the subsequent downstream, such as gene analysis, when the number of tumor cells is small, the background is too much, making it difficult to obtain excellent results.

In a filter method, there remains the problem of how to reduce 1,000 to 10,000 remaining white blood cells.

Thus, white blood cells and tumor cells remaining on the filter are recovered. As the recovery method, a method in which the filter is immersed in a beaker is possible, for example. However, an easy method is to backwash the filter. That is, a liquid is passed backwards through the filter to recover the cells. As the recovery liquid therefor, it is preferable to use a solvent containing a biocompatible polymer.

Examples of solvents containing a biocompatible polymer include the serum and plasma of a mammal. In the case where blood is collected in a blood collection tube containing an anticoagulant and then allowed to stand or centrifuged, the supernatant resulting from the precipitation of blood cell components is plasma. Such plasma contains a coagulation factor. Meanwhile, in the case where blood is collected in a blood collection tube containing no anticoagulant and then allowed to stand, because there is no anticoagulant, the blood cell components coagulate due to a coagulation factor. The supernatant in that case is serum, and serum lacks a coagulation factor.

Among mammals, considering the characteristics or price, it is desirable that the mammal is bovine, equine, or human. In particular, fetal bovine serum is likely to offer excellent characteristics.

Serum contains proteins. Albumin or globulin contained in proteins is adsorbed on the filter surface, whereby blood cell components are prevented from being adsorbed on the filter surface. Proteins are abundantly present in serum, and serum albumin occupies about 50 to 65%.

Albumin contains a large number of amino acids connected, and thus has a large number of amino groups. An amino group firmly coordinates with a noble metal (gold, platinum, palladium).

Particularly in the case of gold, because an oxide film is hardly present, firm binding to albumin is formed even without any special pretreatment.

The concentration of serum or plasma is preferably within a range of 1 to 50%, still more preferably within a range of 5 to 30%, and still more preferably within a range of 10 to 20%.

It is suitable that serum or plasma is diluted with a water-based solvent, and it is desirable that a buffer, such as phosphoric acid, is contained.

Further, it is desirable that the serum or plasma solution contains an anticoagulant of blood. Examples of such anticoagulants include EDTA, heparin, sodium citrate, and sodium fluoride. Among them, EDTA or heparin is desirable.

After a cell suspension containing white blood cells and CTCs diluted with a biocompatible polymer-containing solvent is prepared in this manner, white blood cells are removed.

For the removal of white blood cells, a white blood cell removal method using magnetic beads, which is a conventionally known method, can be used. As such beads, Dynabeads M-450-CD45 (pan-leucocyte) can be mentioned.

Magnetic beads are allowed to react with a CD45 antibody, and then immersed in the cell suspension. At this time, it is suitable that the amount of magnetic beads is about 4 times to 100 times the amount of white blood cells. When the amount of magnetic beads is small, the white blood cell removal efficiency decreases, while when the amount of magnetic beads is large, it leads to increased cost.

Magnetic beads and white blood cells are allowed to react for a predetermined period of time, and then the magnetic beads are removed.

Through the above step, the number of white blood cells can be reduced from the original order of 1,000 to 10,000 (in the case of 7.5 mL) to the order of 10 to 100.

The cell suspension thus obtained is on the level that allows for a downstream.

### Examples

### (Filter 1)

A photosensitive resin composition (PHOTEC RD-1225: 25 µm thick, manufactured by Hitachi Chemical Co., Ltd.) was laminated to one side of a 250-mm-square substrate (MCL-E679F: a substrate prepared by attaching a peelable copper foil to the surface of an MCL, manufactured by Hitachi Chemical Co., Ltd.). The lamination conditions were as follows: roll temperature: 90°C, pressure: 0.3 MPa, conveyor speed: 2.0 m/min.

Next, a glass mask having a light-transmitting part in the shape of a rounded-corner rectangle and designed to have a size of 7.8 (minor pore size) × 100 (major pore size) µm and an aperture ratio of 6.7% was allowed to stand on the photoresist-laminated surface of the substrate. In this example, a glass mask in which rounded-corner rectangles extending in the same direction are aligned in the major-axis and minor-axis directions at constant pitches was used.

Subsequently, in a vacuum of 600 mmHg or less, UV light was applied at an exposure dose of 30 mJ/cm² using a UV irradiation device from above the substrate having the glass mask placed thereon.

Next, the photoresist was developed with a 1.0% aqueous sodium carbonate solution, thereby forming a resist layer having a photoresist in the shape of rectangles vertically standing on the substrate. The copper exposed portion of the resist-deposited substrate was subjected to plating in a nickel plating solution adjusted to pH 4.5 at a temperature of 55°C for about for 20 minutes to give about 20 µm. The composition of the nickel plating solution is shown in Table 1.

**[Table 1]**

| Plating solution composition | Concentration (g/L) |
|---|---|
| Nickel sulfamate | 450 |
| Nickel chloride | 5 |
| Boric acid | 30 |

Next, the obtained nickel plating layer was stripped together with the peelable copper foil of the substrate. The peelable copper foil was removed by chemical lysis with a liquid chemical (MEC Bright SF-5420B, MEC Co., Ltd.) by a stirring treatment at a temperature of 40°C for about 120 minutes, thereby isolating a self-supported film (20 mm × 20 mm) to serve as a metal filter.

Finally, the photoresist remaining in the self-supported film was removed by ultrasonic resist stripping (P3 Poleve, Henkel) at a temperature of 60°C for about 40 minutes, thereby producing a metal filter having through micropores.

Accordingly, a metal filter having no damages, such as wrinkling, bending, or curling, and having through pores with sufficient accuracy was prepared.

Next, the metal filter was immersed in an acidic degreasing solution Z-200 (manufactured by World Metal: trade name) to remove organic matters on the metal filter (40°C, 3 minutes).

After washing with water, a pretreatment of substitution gold plating was performed at 80°C for 10 minutes using a solution prepared by eliminating the gold sulfite, which is a gold supply source, from non-cyanide electroless Au plating HGS-100 (manufactured by Hitachi Chemical: trade name).

Next, the filter was immersed in non-cyanide substitution-type electroless Au plating HGS-100 (manufactured by Hitachi Chemical: trade name) at 80°C for 20 minutes to perform substitution gold plating. The thickness of the substitution gold plating was 0.05 µm.

After washing with water, the filter was immersed in non-cyanide reduction-type electroless Au plating HGS-5400 (manufactured by Hitachi Chemical: trade name) at 65°C for 10 minutes to perform gold plating, washed with water, and then dried. The total thickness of the gold plating was 0.2 µm.

### (Preparation of non-small-cell cancer cell line)

NCI-H358 cells, which are a non-small-cell cancer cell line, were statically cultured in an RPMI-1640 culture medium containing 10% fetal bovine serum (FBS) under conditions of 37°C and 5% CO₂. Cells were separated from the culture dish by a trypsin treatment, recovered, and washed using a phosphate buffer (Phosphate buffered saline, PBS). Subsequently, the NCI-H358 cells were allowed to stand in 10 µM Cell Tracker Red CMTPX (Life Technologies Japan Corporation) at 37°C for 30 minutes and thus stained. Subsequently, the cells were washed with PBS and allowed to stand at 37°C for 3 minutes by a trypsin treatment, whereby cell clusters were dissociated. Subsequently, the trypsin treatment was halted with the culture medium, and the cells were washed with PBS and then suspended in PBS containing 2 mM EDTA and 0.5% bovine serum albumin (BSA) (hereinafter referred to as 2 mM EDTA-0.5% BSA-PBS) to give a suspension for adjustment. Incidentally, PBS is a phosphate buffered saline, and a product code 166-23555 manufactured by Wako Pure Chemical Industries was used. As BSA, a product from SIGMA-ALDRICH (Product Name: Albumin from bovine serum-Lyophilized powder, Bio Reagent for cell culture) was used. As EDTA, 2Na (ethylenediamine-N,N,N',,N'-tetraacetic acid, disodium salt, dihydrate) (product code 345-01865 manufactured by Wako Pure Chemical Industries) was used.

### (Concentration of CTCs in blood sample using magnetic beads)

### (Comparative Example 1)

As a 7.5-ml blood sample, a sample prepared by adding 1,000 of the above tumor cells per 7.5 mL of the blood of a healthy individual collected in an EDTA-2Na-containing vacuum blood collection tube was used. The number of white blood cells in the sample was 4,700,000/ml, that is, 35,250,000/7.5 ml.

An experiment was performed using a CTC recovery device: CT 6000 (manufactured by Hitachi Chemical, trade name), having the filter produced above set to a cartridge. The CTC recovery device includes a flow path for introducing a blood sample or a reagent, and the entrance of the flow path was connected to a reservoir prepared by processing a syringe. The device was designed such that a blood sample or a reagent is successively added to the reservoir, whereby the operations including the capture, staining, and washing of CTCs, etc., can be continuously performed in a simple manner.

First, 1 ml of 2 mM EDTA-10.0% FBS (fetal bovine serum)-PBS (PBS manufactured by Gibco, pH 7.4) (hereinafter referred to as washing liquid) was introduced into the reservoir to fill the filter surface, and then allowed to stand for 10 minutes. Subsequently, liquid delivery was started using a peristaltic pump at a flow rate of 200 µL/min. Subsequently, the Sample 1 was added. About 5 minutes later, 9 mL of the washing liquid was introduced into the reservoir to wash the cells.

Next, a solution of 4% PFA in PBS was placed in the cartridge, and the cells were immersed for 15 minutes. After washing, a solution of 0.2% TritonX in the washing liquid was placed in the cartridge, and the cells were immersed for 15 minutes.

Another 10 minutes later, the pump flow rate was changed to 20 µL/min. 600 µL of a cell staining solution (Hoechst 33342: 30 µl, Wash buffer: 300 ml) was introduced into the reservoir, and the tumor cells or white blood cells on the filter were fluorescently stained. After cells captured on the filter were stained for 30 minutes, 1 mL of 2 mM EDTA-0.5% BSA-PBS was introduced into the reservoir to wash the cells.

Subsequently, the filter was observed using a fluorescence microscope (BX61, Olympus Corporation) equipped with a computer-controlled electrical stage and a cooled digital camera (DP70, Olympus Corporation), and the number of tumor cells and the number of white blood cells on the filter were counted.

In order to observe the fluorescence from Hoechst 33342 and CellTracker Red CMTPX, images were acquired using WU and WIG filters (Olympus Corporation), respectively. As the image acquisition and analysis software, Lumina Vision (Mitani Corporation) was used. The results are shown in Table 2. Cell recovery rate (%) = the number of tumor cells recovered on the filter/the number of tumor cells mixed to the blood sample × 100%. The number of white blood cells was calculated by subtracting the number of tumor cells from the number of cells stained with Hoechst 33342. In this manner, the experiment of Comparative Example 1 was performed.

### (Comparative Example 2)

An experiment was performed in the same manner as in Comparative Example 1, except that the initial amount of blood was changed from 7.5 ml to 15 ml.

### (Comparative Example 3)

An experiment was performed in the same manner as in Comparative Example 1, except that the initial amount of blood was changed from 7.5 ml to 30 ml.

### (Example 1)

As a 7.5-ml blood sample, a sample prepared by adding 1,000 of the above tumor cells per 7.5 mL of the blood of a healthy individual collected in an EDTA-2Na-containing vacuum blood collection tube was used. The number of white blood cells in the sample was 4,700,000/ml, that is, 35,250,000/7.5 ml.

Blood filtration was performed using a CTC recovery device: CT 6000 (manufactured by Hitachi Chemical, trade name), having the filter produced above set to a cartridge. The CTC recovery device includes a flow path for introducing a blood sample or a reagent, and the entrance of the flow path was connected to a reservoir prepared by processing a syringe. The device was designed such that a blood sample or a reagent is successively added to the reservoir, whereby the operations including the capture, staining, and washing of CTCs, etc., can be continuously performed in a simple manner.

First, 1 ml of 2 mM EDTA-10.0% FBS (fetal bovine serum)-PBS (PBS manufactured by Gibco, pH 7.4) (hereinafter referred to as washing liquid) was introduced into the reservoir to fill the filter surface, and then allowed to stand for 10 minutes. Subsequently, liquid delivery was started using a peristaltic pump at a flow rate of 200 µL/min. Subsequently, the blood sample was added. About 5 minutes later, 9 mL of the washing liquid was introduced into the reservoir to wash the cells.

Next, a washing liquid was passed backwards through the filter to recover cells on the filter (backwashing), thereby giving 10 ml of a cell-containing suspension.

1 ml of a dispersion of 4.0 × 10⁵ Dynabeads CD45, which are magnetic beads provided with a CD45 antibody (0.1% BSA, 0.02% Sodium azide, PBS pH 7.4) was added and allowed to react for a predetermined period of time, and then the magnetic beads were removed using a magnet, thereby giving a suspension 1 containing remaining tumor cells.

A CTC recovery operation was performed using a CTC recovery device: CT 6000 (manufactured by Hitachi Chemical, trade name), having the filter produced above set to a cartridge. The CTC recovery device includes a flow path for introducing a blood sample or a reagent, and the entrance of the flow path was connected to a reservoir prepared by processing a syringe. The device was designed such that a blood sample or a reagent is successively added to the reservoir, whereby the operations including the capture, staining, and washing of CTCs, etc., can be continuously performed in a simple manner.

First, 1 ml of 2 mM EDTA-10.0% FBS (fetal bovine serum)-PBS (PBS manufactured by Gibco, pH 7.4) (hereinafter referred to as washing liquid) was introduced into the reservoir to fill the filter surface, and then allowed to stand for 10 minutes. Subsequently, liquid delivery was started using a peristaltic pump at a flow rate of 200 µL/min. Subsequently, the suspension 1 was added. About 5 minutes later, 9 mL of the washing liquid was introduced into the reservoir to wash the cells.

Next, a solution of 4% PFA in PBS was placed in the cartridge, and the cells were immersed for 15 minutes.

After washing, a solution of 0.2% TritonX in the washing liquid was placed in the cartridge, and the cells were immersed for 15 minutes.

Another 10 minutes later, the pump flow rate was changed to 20 µL/min. 600 µL of a cell staining solution (Hoechst 33342: 30 µl, Wash buffer: 300 ml) was introduced into the reservoir, and the tumor cells or white blood cells on the filter were fluorescently stained. After cells captured on the filter were stained for 30 minutes, 1 mL of 2 mM EDTA-0.5% BSA-PBS was introduced into the reservoir to wash the cells.

Subsequently, the filter was observed using a fluorescence microscope (BX61, Olympus Corporation) equipped with a computer-controlled electrical stage and a cooled digital camera (DP70, Olympus Corporation), and the number of tumor cells and the number of white blood cells on the filter were counted.

In order to observe the fluorescence from Hoechst 33342 and CellTracker Red CMTPX, images were acquired using WU and WIG filters (Olympus Corporation), respectively. As the image acquisition and analysis software, Lumina Vision (Mitani Corporation) was used. The results are shown in Table 2. Cell recovery rate (%) = the number of tumor cells recovered on the filter/the number of tumor cells mixed to the blood sample × 100%. The number of white blood cells was calculated by subtracting the number of tumor cells from the number of cells stained with Hoechst 33342.

### (Example 2)

The experiment of Example 2 was performed in the same manner as in Example 1, except that the initial amount of blood was changed from 7.5 ml to 15 ml.

### (Example 3)

The experiment of Example 3 was performed in the same manner as in Example 1, except that the initial amount of blood was changed from 7.5 ml to 30 ml.

The results of the examples and comparative examples are shown in Table 2.

**[Table 2]**

| Example | The number of remaining white blood cells | Tumor cell recovery rate |
|---|---|---|
| Comparative Example 1 | 1862 | 94.9% |
| Comparative Example 2 | 4763 | 89.8% |
| Comparative Example 3 | 9865 | 88.1% |
| Example 1 | 18 | 89.2% |
| Example 2 | 48 | 86.4% |
| Example 3 | 99 | 79.9% |

In Comparative Examples 1 to 3, in which the experiments were performed without using magnetic beads, the number of residual white blood cells is large, which is unsuitable for a downstream. In Examples 1 to 3, white blood cells in the cells of comparative examples were reduced using magnetic beads, and then filtered. In each case, white blood cells were reduced, which is applicable to a downstream. However, when the number of white blood cells before performing magnetic beads is large, the number of remaining white blood cells tends to increase accordingly. Further, the tumor cell recovery rate decreases. It is preferable that the number of white blood cells after filter filtration is 10,000 or less.

As shown above, by combining the filter shown in the prevent invention with the removal of white blood cells by an antigen-antibody reaction, the characteristics are improved over conventional filters, allowing for a downstream.

### Reference Signs List

1: MCL, 2: peelable copper foil, 2': copper plate, 3: photoresist, 3a: exposed portion of photoresist (exposed area), 3b: developed portion of photoresist (unexposed area), 4: photomask, 5: plating layer, 6: through pore, 7: gold plating layer.

## Claims

1. A method for capturing rare cells in blood, comprising isolating rare cells in blood from blood,
the method including a step of removing white blood cells using beads having magnetic properties, after blood filtration using a filter having a surface made of gold, platinum, palladium, or an alloy thereof, and
the method comprising, before the blood filtration, a step of immersing the filter in an aqueous solution containing the serum or plasma of a mammal.

2. The method for capturing rare cells in blood according to claim 1, wherein the number of white blood cells in a liquid containing the rare cells in blood obtained after the blood filtration is 10,000 or less.

3. The method for capturing rare cells in blood according to claim 1, wherein the serum or plasma of a mammal is of bovine, equine, or human origin.

4. The method for capturing rare cells in blood according to claim 1, wherein the serum or plasma of a mammal is from a bovine fetus.

5. The method for capturing rare cells in blood according to any one of claims 1 to 4, wherein the concentration of the serum or plasma of a mammal in the aqueous solution is within a range of 1% to 50%.

6. The method for capturing rare cells in blood according to any one of claims 1 to 5, wherein the aqueous solution of serum or plasma contains a phosphate buffer as a main component.

7. The method for capturing rare cells in blood according to any one of claims 1 to 6, wherein the aqueous solution of serum or plasma contains an anticoagulant.

8. The method for capturing rare cells in blood according to claim 7, wherein the anticoagulant is EDTA, heparin, sodium citrate, or sodium fluoride.

9. The method for capturing rare cells in blood according to any one of claims 1 to 8, further comprising, after the blood filtration and before the step of removing white blood cells, a step of washing blood cells with an aqueous solution containing the serum or plasma of a mammal.

10. The method for capturing rare cells in blood according to any one of claims 1 to 9, wherein the serum or plasma of a mammal is of bovine, equine, or human origin.

11. The method for capturing rare cells in blood according to any one of claims 1 to 9, wherein the serum of a mammal is fetal bovine serum or plasma.

12. The method for capturing rare cells in blood according to any one of claims 1 to 11, wherein the filter contains nickel as a main component.

13. The method for capturing rare cells in blood according to any one of claims 1 to 11, wherein the filter contains copper as a main component.

14. The method for capturing rare cells in blood according to any one of claims 1 to 11, wherein the filter contains palladium as a main component.

15. The method for capturing rare cells in blood according to any one of claims 1 to 14, wherein an outermost layer of the filter is gold plating.

16. The method for capturing rare cells in blood according to any one of claims 1 to 14, wherein the surface of gold, platinum, palladium or an alloy thereof is a plating having a thickness of 0.05 µm to 1 µm.

17. The method for capturing rare cells in blood according to any one of claims 1 to 16, wherein the rare cells in blood are tumor cells.

18. The method for capturing rare cells in blood according to any one of claims 1 to 17, wherein the filter has through pores whose opening shape is a circle, an ellipse, a rounded-corner rectangle, a rectangle, or a square.

19. The method for capturing rare cells in blood according to any one of claims 1 to 18, wherein the filter has through pores whose opening shape is at least one of a rectangle and a rounded-corner rectangle, the minor length thereof being 5 µm to 15 µm.

20. The method for capturing rare cells in blood according to any one of claims 1 to 19, wherein the filter has a thickness of 3 µm to 50 µm.

## Patentansprüche

1. Verfahren zum Abfangen seltener Zellen in Blut, umfassend Isolieren seltener Zellen in Blut aus Blut,
wobei das Verfahren einen Schritt umfasst, bei dem weiße Blutzellen unter Verwendung von Kügelchen mit magnetischen Eigenschaften entfernt werden nach Blutfiltration unter Verwendung eines Filters mit einer aus Gold, Platin, Palladium oder eine Legierung davon gebildeten Oberfläche, und
wobei das Verfahren vor der Blutfiltration einen Schritt umfasst, bei dem der Filter in eine wässrige Lösung getaucht wird, die das Serum oder Plasma eines Säugers enthält.

2. Verfahren zum Abfangen seltener Zellen in Blut gemäß Anspruch 1, wobei die Anzahl an weißen Blutzellen in einer die seltenen Zellen enthaltenden nach der Blutfiltration erhaltenen Flüssigkeit 10.000 oder weniger beträgt.

3. Verfahren zum Abfangen seltener Zellen in Blut gemäß Anspruch 1,
wobei das Serum oder das Plasma eines Säugers von Rinder-, Pferde-oder humaner Herkunft ist.

4. Verfahren zum Abfangen seltener Zellen in Blut gemäß Anspruch 1, wobei das Serum oder Plasma eines Säugers von einem Rinderfötus stammt.

5. Verfahren zum Abfangen seltener Zellen in Blut gemäß einem der Ansprüche 1 bis 4,
wobei die Konzentration des Serums oder des Plasmas eines Säugers in der wässrigen Lösung innerhalb eines Bereichs von 1 % bis 50 % liegt.

6. Verfahren zum Abfangen seltener Zellen in Blut gemäß einem der Ansprüche 1 bis 5,
wobei die wässrige Serum- oder Plasmalösung ein Phosphatspuffer als eine Hauptkomponente enthält.

7. Verfahren zum Abfangen seltener Zellen in Blut gemäß einem der Ansprüche 1 bis 6,
wobei die wässrige Serum-oder Plasmalösung ein Antikoagulans enthält.

8. Verfahren zum Abfangen seltener Zellen in Blut gemäß einem der Ansprüche 1 bis 7,
wobei das Antikoagulans EDTA, Heparin, Natriumcitrat oder Natriumfluorid ist.

9. Verfahren zum Abfangen seltener Zellen in Blut gemäß einem der Ansprüche 1 bis 8,
das nach der Blutfiltration und vor dem Schritt des Entfernens weißer Blutzellen weiterhin einen Schritt umfasst, bei dem Blutzellen mit einer wässrigen Lösung gewaschen werden, die das Serum oder Plasma eines Säugers enthält.

10. Verfahren zum Abfangen seltener Zellen in Blut gemäß einem der Ansprüche 1 bis 9,
wobei das Serum oder Plasma eines Säugers von Rinder-, Pferde- oder humaner Herkunft ist.

11. Verfahren zum Abfangen seltener Zellen in Blut gemäß einem der Ansprüche 1 bis 9,
wobei das Serum eines Säugers fötales Rinderserum oder Plasma ist.

12. Verfahren zum Abfangen seltener Zellen in Blut gemäß einem der Ansprüche 1 bis 11,
wobei der Filter Nickel als eine Hauptkomponente enthält.

13. Verfahren zum Abfangen seltener Zellen in Blut gemäß einem der Ansprüche 1 bis 11,
wobei der Filter Kupfer als eine Hauptkomponente enthält.

14. Verfahren zum Abfangen seltener Zellen in Blut gemäß einem der Ansprüche 1 bis 11,
wobei der Filter Palladium als eine Hauptkomponente enthält.

15. Verfahren zum Abfangen seltener Zellen in Blut gemäß einem der Ansprüche 1 bis 14,
wobei eine äußerste Schicht des Filters Goldbeschichtung ist.

16. Verfahren zum Abfangen seltener Zellen in Blut gemäß einem der Ansprüche 1 bis 14, wobei die Oberfläche aus Gold, Platin, Palladium oder eine Legierung davon eine Beschichtung mit einer Dicke von 0,05 µm bis 1 µm ist.

17. Verfahren zum Abfangen seltener Zellen in Blut gemäß einem der Ansprüche 1 bis 16, wobei die seltenen Zellen im Blut Tumorzellen sind.

18. Verfahren zum Abfangen seltener Zellen in Blut gemäß einem der Ansprüche 1 bis 17, wobei der Filter durchgehende Poren aufweist, deren Öffnungsform ein Kreis, eine Ellipse, ein Rechteck mit abgerundeten Ecken, ein Rechteck oder ein Quadrat ist.

19. Verfahren zum Abfangen seltener Zellen in Blut gemäß einem der Ansprüche 1 bis 18, wobei der Filter durchgehende Poren aufweist, deren Öffnungsform wenigstens eine aus einem Rechteck mit abgerundeten Ecken und einem Rechteck ist, wobei die kleine Länge davon 5 µm bis 15 µm beträgt.

20. Verfahren zum Abfangen seltener Zellen in Blut gemäß einem der Ansprüche 1 bis 19, wobei der Filter eine Dicke von 3 µm bis 50 µm aufweist.

## Revendications

1. Procédé de capture de cellules rares dans le sang, comprenant l'isolation de cellules rares dans le sang à partir de sang,
le procédé comportant une étape d'élimination de cellules sanguines blanches en utilisant des billes ayant des propriétés magnétiques, après filtration du sang en utilisant un filtre ayant une surface faite d'or, de platine, de palladium, ou d'un alliage de ceux-ci, et
le procédé comprenant, avant la filtration du sang, une étape d'immersion du filtre dans une solution aqueuse contenant le sérum ou plasma d'un mammifère.

2. Le procédé de capture de cellules rares dans le sang selon la revendication 1, dans lequel le nombre de cellules sanguines blanches dans un liquide contenant les cellules rares dans le sang obtenu après la filtration du sang est de 10000 ou moins.

3. Le procédé de capture de cellules rares dans le sang selon la revendication 1, dans lequel le sérum ou plasma d'un mammifère est d'origine humaine, équine, ou bovine.

4. Le procédé de capture de cellules rares dans le sang selon la revendication 1, dans lequel le sérum ou plasma d'un mammifère provient d'un foetus bovin.

5. Le procédé de capture de cellules rares dans le sang selon l'une quelconque des revendications 1 à 4, dans lequel la concentration du sérum ou plasma d'un mammifère dans la solution aqueuse est dans un domaine de 1% à 50%.

6. Le procédé de capture de cellules rares dans le sang selon l'une quelconque des revendications 1 à 5, dans lequel la solution aqueuse de sérum ou plasma contient un tampon phosphate en tant que composant principal.

7. Le procédé de capture de cellules rares dans le sang selon l'une quelconque des revendications 1 à 6, dans lequel la solution aqueuse de sérum ou plasma contient un anticoagulant.

8. Le procédé de capture de cellules rares dans le sang selon l'une quelconque des revendications 1 à 7, dans lequel l'anticoagulant est l'EDTA, l'héparine, le citrate de sodium, ou le fluorure de sodium.

9. Le procédé de capture de cellules rares dans le sang selon l'une quelconque des revendications 1 à 8, comprenant en outre, après la filtration du sang et avant l'étape d'élimination des cellules sanguines blanches, une étape de lavage des cellules sanguines avec une solution aqueuse contenant le sérum ou plasma d'un mammifère.

10. Le procédé de capture de cellules rares dans le sang selon l'une quelconque des revendications 1 à 9, dans lequel le sérum ou plasma d'un mammifère est d'origine humaine, équine ou bovine.

11. Le procédé de capture de cellules rares dans le sang selon l'une quelconque des revendications 1 à 9, dans lequel le sérum d'un mammifère est du plasma ou sérum de foetus bovin.

12. Le procédé de capture de cellules rares dans le sang selon l'une quelconque des revendications 1 à 11, dans lequel le filtre contient du nickel en tant que composant principal.

13. Le procédé de capture de cellules rares dans le sang selon l'une quelconque des revendications 1 à 11, dans lequel le filtre contient du cuivre en tant que composant principal.

14. Le procédé de capture de cellules rares dans le sang selon l'une quelconque des revendications 1 à 11, dans lequel le filtre contient du palladium en tant que composant principal.

15. Le procédé de capture de cellules rares dans le sang selon l'une quelconque des revendications 1 à 14, dans lequel une couche la plus extérieure du filtre est un placage d'or.

16. Le procédé de capture de cellules rares dans le sang selon l'une quelconque des revendications 1 à 14, dans lequel la surface d'or, de platine, de palladium et un alliage de ceux-ci est un placage ayant une épaisseur de 0,05 µm à 1 µm.

17. Le procédé de capture de cellules rares dans le sang selon l'une quelconque des revendications 1 à 16, dans lequel les cellules rares dans le sang sont des cellules tumorales.

18. Le procédé de capture de cellules rares dans le sang selon l'une quelconque des revendications 1 à 17, dans lequel le filtre des pores traversantes dans la forme d'ouverture est un cercle, un ellipse, un rectangle à coins arrondis, un rectangle, ou un carré.

19. Le procédé de capture de cellules rares dans le sang selon l'une quelconque des revendications 1 à 18, dans lequel le filtre a des pores traversantes dont la forme d'ouverture est au moins l'une d'un rectangle et d'un rectangle à coins arrondis, la longueur mineure de celui-ci étant de 5 µm à 15 µm.

20. Le procédé de capture de cellules rares dans le sang selon l'une quelconque des revendications 1 à 19, dans lequel le filtre a une épaisseur de 3 µm à 50 µm.
